Europäisches Patentamt

European Patent Office

Office européen des brevets

(19)

(11) Veröffentlichungsnummer: **0 269 823**

**A1**

(12) # EUROPÄISCHE PATENTANMELDUNG

(21) Anmeldenummer: 87115372.2

(22) Anmeldetag: 21.10.87

(51) Int. Cl.4: **A61B 5/08** , G01F 1/68 , G01P 5/12

(30) Priorität: **20.11.86 DE 3639666**

(43) Veröffentlichungstag der Anmeldung:
**08.06.88 Patentblatt 88/23**

(84) Benannte Vertragsstaaten:
**DE FR GB SE**

(71) Anmelder: **Drägerwerk Aktiengesellschaft
Moislinger Allee 53-55
D-2400 Lübeck 1(DE)**

(72) Erfinder: **Kiske, Siegfried, Dr. Dipl.-Ing.
Am Sonnenberg 1e
D-2401 Gross Grönau(DE)**

(54) Verfahren zur Bestimmung einer Messgrösse eines strömenden Mediums und Messschaltung hierzu.

(57) Ein Verfahren zur Bestimmung einer Meßgröße eines strömenden Mediums, insbesondere der Strömungsgeschwindigkeit und/oder der Stoffeigenschaften, bei dem die Temperaturänderung eines beheizten Meßsensors in einer elektronischen Meßschaltung zur Bestimmung der Meßgröße ausgewertet wird, soll hinsichtlich der Meßgenauigkeit, insbesondere im Bereich geringer Strömungsgeschwindigkeiten, verbessert werden. Dies wird gemäß der Erfindung dadurch erreicht, daß der Meßsensor innerhalb wenigstens eines Meßabschnittes mit einem Aufheizimpuls aufgeheizt wird, und daß als Meßgröße die Zeit bestimmt wird, nach der der Meßsensor auf mindestens eine vorgegebene Temperatur abgekühlt ist.

EP 0 269 823 A1

Beschreibung

D r ä g e r w e r k  Aktiengesellschaft
Moislinger Allee 53-55, 2400 Lübeck, DE

Verfahren zur Bestimmung einer Meßgröße eines
strömenden Mediums und Meßschaltung hierzu

Die Erfindung betrifft ein Verfahren zur Bestimmung einer
Meßgröße eines strömenden Mediums, insbesondere der
Strömungsgeschwindigkeit und/oder der Stoffeigenschaften,
bei dem die Temperaturänderung eines beheizten Meßsensors
in einer elektronischen Meßschaltung zur Bestimmung der
Meßgröße ausgewertet wird.

Durch die DE-OS 29 33 116 ist eine Einrichtung zur Messung
des Atemluftstromes bekannt, bei der zwei im Abstand
hintereinander liegende Meßsensoren angewendet werden,
welche durch einen Regelkreis auf konstante Temperatur
aufgeheizt werden, wobei die hierzu erforderliche
Heizenergie die Meßgröße darstellt.

Eine andere vorbekannte Meßeinrichtung zur Bestimmung der
Wärmeleitfähigkeit eines Gasgemisches, die in der
DE-PS 25 05 669 beschrieben ist, ermöglicht die Bestimmung
der Strömungsgeschwindigkeit und der Gaszusammensetzung.
Hierzu wird ein Widerstands-Meßsensor durch elektrischen
Strom auf eine bestimmte Temperatur erwärmt, die durch
eine entsprechende Regelschaltung eingehalten wird. Der
jeweils im Meßsensor auftretende Strom zur Aufrechterhaltung
der konstanten Temperatur bildet die Meßgröße. Die
Gasführung in einem Strömungskanal erfolgt intermittierend,
wobei Strömungsintervalle und strömungsfreie Intervalle
in gleichen Abständen wechseln. Im Strömungsintervall
bildet die Meßgröße ein Maß für die Wärmeleitfähigkeit und
die Strömungsgeschwindigkeit des Mediums. Im strömungsfreien
Intervall wird die Wärmeleitfähigkeit des Mediums z.B.
eines Gasgemisches bestimmt.

Die Erfindung geht von der Aufgabenstellung aus, die bekannten Nachteile der Konstanttemperaturhitzdrahtanemometrie zu vermeiden und insbesondere die Meßgenauigkeit bei Messungen der Strömungsgeschwindigkeit und/oder der Stoffeigenschaften von Fluiden, insbesondere bei geringen Strömungsgeschwindigkeiten, zu verbessern.

Diese Aufgabe wird erfindungsgemäß dadurch gelöst, daß der Meßsensor innerhalb wenigstens eines Meßabschnittes mit einem Aufheizimpuls aufgeheizt wird, und daß als Meßgröße die Zeit bestimmt wird, nach der der Meßsensor auf mindestens eine vorgegebene Temperatur abgekühlt ist.

Im Gegensatz zum vorbekannten Konstanttemperatur- Verfahren wird die Meßaufgabe von einer aufwendigen und vielen Störeffekten ausgesetzten Temperaturmessung auf eine genauer durchführbare Zeitmessung übertragen. Störende Nebeneffekte, wie z.B. die Wärmeleitung vom eigentlichen Meßsensor an die Zuführungsleitungen oder Sensorhalterungen, sind nur von geringer Wirkung. Außerdem wird durch die impulsweise Aufheizung deutlich weniger Strom verbraucht.

In der praktischen Ausführung wird jeweils eine Reihe von Aufheizimpulsen verwendet, wobei in der einen zweckmäßigen Ausführung des Verfahrens der Aufheizimpuls jeweils dann ausgelöst wird, wenn der Meßsensor eine vorgegebene Temperatur erreicht, so daß sich bei schwankenden Meßgrößen, beispielsweise unterschiedlichen Strömungsgeschwindigkeiten, variable Impulsabstände ergeben. Eine andere gegebenenfalls zweckmäßige Ausführung des Verfahrens kann darin bestehen, daß eine Reihe von Aufheizimpulsen mit gleichem Abstand verwendet wird, wobei der Impulsabstand so groß gewählt werden muß, daß der nachzuweisende Grenzwert der Meßgröße, beispielsweise die geringste Strömungsgeschwindigkeit, noch innerhalb eines
Meßabschnittes liegt.

Es hat sich als günstig erwiesen, die Aufheizzeit, d.h.
die Dauer der Aufheizimpulse, sehr kurz im Vergleich zur
Länge der Meßabschnitte zu machen. Zweckmäßigerweise liegt
die Aufheizzeit unterhalb 150 $\mu$sec, während die Längen
der Meßabschnitte vorteilhaft zwischen 1 und 50 msec
festgelegt werden. Durch die sehr kurze Aufheizzeit werden
Beeinträchtigungen der Wärmeableitung des aufgeheizten
Sensors durch störende Nebeneffekte weiter reduziert und
sogar vermieden, weil das Abkühlen des Sensors nunmehr im
wesentlichen nur durch den konvektiven Wärmetransport an
das umströmende Medium bestimmt ist.

Durch periodisches Aufheizen und Abkühlen kann bei
bekannten Stoffeigenschaften des strömenden Mediums in
eindeutiger Weise auf die Strömungsgeschwindigkeit
geschlossen werden. Da durch theoretische und
experimentelle Untersuchungen nachgewiesen ist, daß das
Abklingen der Temperatur infolge erzwungener Konvektion
exponentiell erfolgt, ist der Betrag des Exponenten ein
Maß der Strömungsgeschwindigkeit.

Zur Simultanbestimmung von Strömungsgeschwindigkeit und
Stoffeigenschaften sind im allgemeinen mehrere linear
unabhängige Messungen erforderlich, was z.B. durch
gleichzeitiges Bestimmen des Abklingverhaltens von
mehreren Meßsensoren unterschiedlicher Ausbildung erfolgen
kann. Dieses vorbekannte Verfahren bedingt einen komplexen
Aufbau der Meßsensoren sowie lange Rechenzeiten bei
prozessorgesteuerter Auswertung. In vielen Anwendungsfällen
ist außerdem eine fehlerfreie Messung der
Strömungsgeschwindigkeit bei unterschiedlicher
Zusammensetzung des strömenden Mediums notwendig. Dies erfordert eine ständige Kalibrationsmessung zur Kompensation des Einflusses der sich ändernden Zusammensetzung des strömenden Mediums. Eine solche Kompensationsmessung zur Ermittlung des Einflusses der Gaseigenschaften kann bei einem definierten Geschwindigkeitszustand, im allgemeinen beim Nullwert der Strömungsgeschwindigkeit, durchgeführt werden. Ein solches intermittierendes Meßverfahren ist in der DE-PS 25 05 669 beschrieben. Eine wichtige Voraussetzung für diese Messung bildet die Kenntnis des exakten Nullwertes der Strömungsgeschwindigkeit.

In weiterer Ausgestaltung der Erfindung kann zur Bestimmung des Strömungs-Nullwertes die Größe der Abweichung vom exponentiellen Verlauf der Temperaturkurve festgestellt werden, wobei der Nullwert jeweils dann vorliegt, wenn der Betrag der Abweichung vom exponentiellen Verlauf seinen experimentell bestimmbaren Grenzwert erreicht. Der Betrag der Abweichung vom exponentiellen Abklingverhalten ist bei der Geschwindigkeit Null maximal, er nimmt mit wachsender Geschwindigkeit ab und verschwindet schließlich bei einer Strömungsgeschwindigkeit, die größer ist als die Diffusionsgeschwindigkeit durch Wärmeleitung, so daß die Abkühlkurve wieder in den Exponentialverlauf übergeht.

Als Maß für die Größe der Abweichung vom exponentiellen Verlauf der Temperaturkurve können vorteilhaft die Temperaturen gewählt werden, für die sich nach dem exponentiellen Verlauf der Abkühlungs-Temperaturkurve gleiche Zeitdifferenzen $\Delta t_1 = t_2 - t_1$ und $\Delta t_2 = t_3 - t_2$ ergeben würden, wobei das Verhältnis der tatsächlich gemessenen Zeitdifferenzen $\Delta t_1$ und $\Delta t_2$ zur Erreichung dieser Temperaturen bestimmt wird und der Grenzwert dieses Verhältnisses den Nullwert der Strömung festlegt.

Wenn also durch Messung des Abklingzeitverhältnisses $\frac{\Delta t_1}{\Delta t_2}$ feststeht, daß der Grenzwert dieses Verhältnisses, und damit der Strömungsnullwert erreicht wird, ist der Verlauf der Abkühlungs-Temperaturkurve ausschließlich durch freie Konvektion bestimmt. Bei festgelegten Sensoreigenschaften hängt die Abkühlgeschwindigkeit dann nur noch von den Stoffeigenschaften des strömenden Mediums ab. Damit lassen sich als Meßgrößen bestimmte Stoffeigenschaften, beispielsweise die Gaszusammensetzung feststellen. Ein solcher Meßwert kann dann u.U. als Referenzwert zum Ausgleich des Einflusses schwankender Zusammensetzungen des Strömungsmediums auf die Geschwindigkeitsmessung benutzt werden.

Eine zweckmäßige Meßschaltung zur Durchführung des Verfahrens kann so aufgebaut sein, daß ein elektronisches Schaltelement vorgesehen ist, welches mit einem Pulsgenerator verbunden ist und den Meßsensor in seiner einen Schaltstellung mit einer Heizspannungsquelle und in seiner anderen Schaltstellung mit einer Widerstandsmeßanordnung verbindet, daß die Umschaltung zwischen beiden Schaltzuständen taktweise in gleichem Abstand bzw. dann erfolgt, wenn der Meßsensor einen vorgegebenen Widerstandswert erreicht, und daß zur Bestimmung des zeitlichen Abstandes zwischen dem Aufheizimpuls und dem Erreichen des vorgegebenen Widerstandswertes Komparatoren vorgesehen sind.

In Abhängigkeit von der Impulsfolge der Heizspannungsquelle kann vorteilhaft jeweils eine Reihe von in gleichem Abstand liegenden Abstandsimpulsen erzeugt werden, und die zu bestimmende Meßgröße läßt sich in diesem Falle durch Abzählen der Anzahl der Abstandsimpulse ermitteln, welche zwischen dem Aufheizimpuls und dem Erreichen des vorgegebenen Widerstandswertes liegen.

In der Zeichnung soll die Durchführung des erfindungsgemäßen Verfahrens und der Aufbau einer entsprechenden Meßschaltung erläutert werden; es zeigen:

Fig. 1 eine Reihe von Aufheizimpulsen,

Fig. 2 eine Meßschaltung zur Durchführung des Verfahrens,

Fig. 3 eine Darstellung von in der Meßschaltung auftretenden Signalen,

Fig. 4 eine Abklingkurve bei der Geschwindigkeit Null.

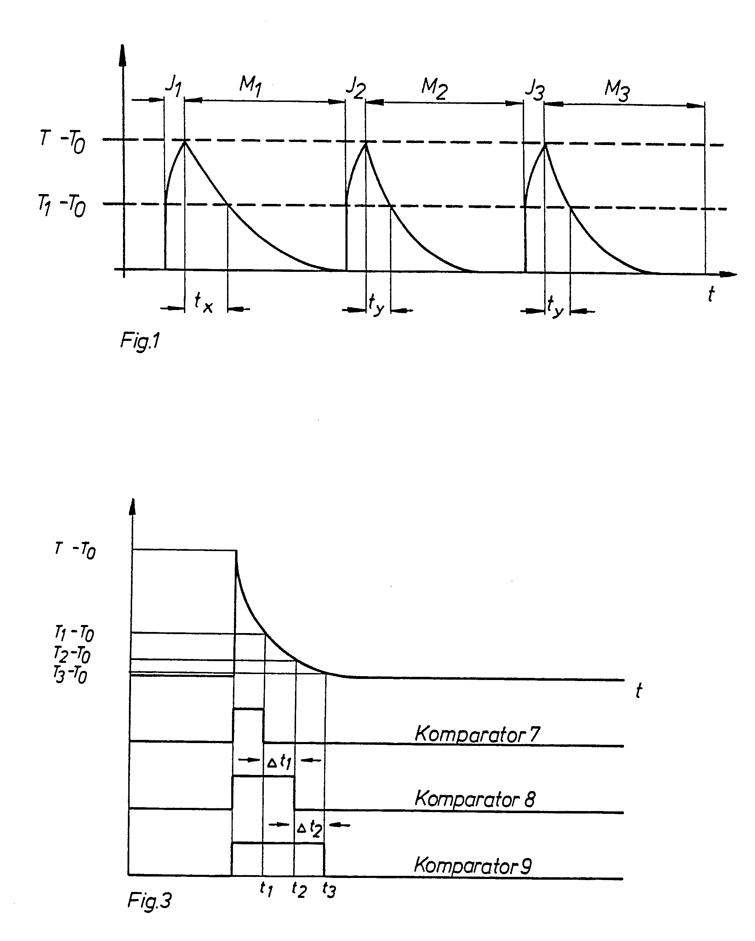

In Fig. 1 ist eine Reihe von in gleichem Abstand aufeinander folgenden Aufheizimpulsen $J_1$, $J_2$, $J_3$ dargestellt, welche jeweils am Anfang der Meßabschnitte $M_1$, $M_2$, $M_3$ liegen. Durch diese Aufheizimpulse $J_1$, $J_2$, $J_3$ wird der Meßsensor auf eine Temperatur $T-T_0$ oberhalb der Umgebungstemperatur $T_0$, beispielsweise auf $300^\circ C$ aufgeheizt. Nach Beendigung des Aufheizimpulses klingt die ursprüngliche Aufheiztemperatur $T-T_0$ bis auf den vorgegebenen Wert $T_1-T_0$ ab. Die hierfür benötigte Zeit $t_x$ ist von der Strömungsgeschwindigkeit am Meßsensor abhängig. Aus den durch Zeitmessung ermittelten Zeiten $t_x$, $t_y$ kann mit hoher Genauigkeit auf die Strömungsgeschwindigkeit des den Meßsensor umströmenden Mediums geschlossen werden. $t_x$ bedeutet eine geringere Strömungsgeschwindigkeit im Meßabschnitt $M_1$, während in den Meßabschnitten $M_2$ und $M_3$ jeweils gleiche, aber höhere Strömungsgeschwindigkeiten, ausgedrückt durch $t_y$, vorliegen.

Der innere thermische Widerstand des Sensors soll zur einwandfreien Ausführung der Messungen wesentlich kleiner sein als der Widerstand für den Wärmeübergang an das umgebende Medium. Dies wird bei Gasen mit hoher Genauigkeit dann erreicht, wenn der Meßsensor aus üblichem Material, beispielsweise aus einem Platin- oder Wolframdraht mit einem Durchmesser< 0,05 mm besteht.

Ist die Dauer der Aufheizimpulse $J_1$... ausreichend kurz
gewählt, bleibt die maximale Temperatur des Meßsensors
von der Strömungsgeschwindigkeit und von Stoffeigenschaften
unabhängig. Die Gesamtlänge der Meßabschnitte $M_1$... soll
ausreichend lang sein, so daß immer sichergestellt wird, daß
der Meßsensor beim Abkühlen die vorgegebene Temperatur
$T_1-T_0$ annimmt.

Bei dem Ausführungsbeispiel nach Fig. 1 sind jeweils gleich
lange Meßabschnitte $M_1$, $M_2$, $M_3$ angegeben. Dies bedeutet,
daß die Aufheizung erst nach Ablauf des vollen
Meßabschnittes erneut einsetzt. Für manche Anwendungszwecke
(Erhöhung der Meßfrequenz) erscheint es jedoch
zweckmäßiger, bei variabler Länge der Meßabschnitte
jeweils dann den Aufheizimpuls auszulösen, wenn die
vorgegebene Temperatur $T_1 - T_0$ erreicht wird.

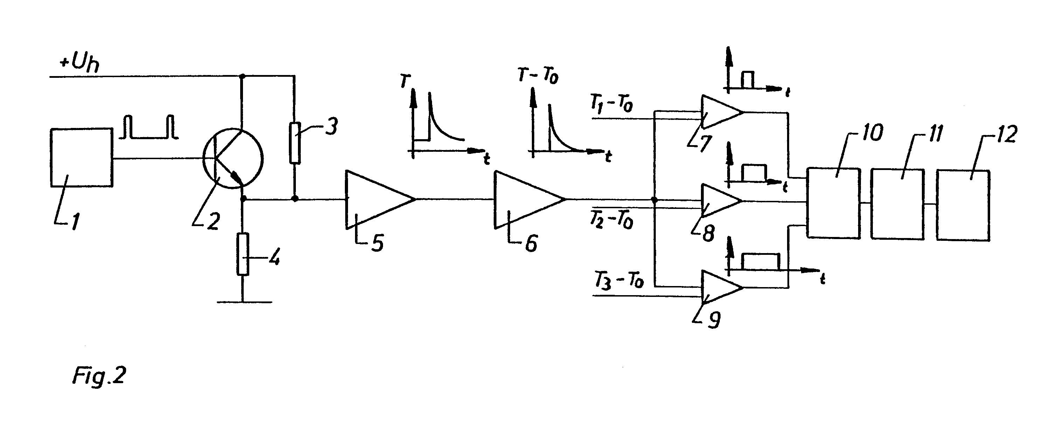

Die Meßschaltung nach Fig. 2 enthält einen Pulsgenerator 1,
mit dem über einen Transistorschalter 2 die in Fig. 1
dargestellte Impulsfolge mit gleicher Länge der Meßabschnitte
und die Auslösung der Aufheizimpulse $J_1$, $J_2$... gesteuert
werden. Der Pulsgenerator 1 schließt den Transistorschalter 2
bei jedem Impuls, so daß in dieser Schaltstellung die
Heizspannung $U_h$ an den Meßsensor 4 angelegt wird. Während
der Pulspause sperrt der Transistorschalter 2, und in dieser
Schaltstellung wird die Messung des Widerstandes am
Meßsensor durchgeführt. Der über den Widerstand 3 in den
Meßsensor 4 eingespeiste Meßstrom ist so groß gewählt,
daß einerseits keine merkliche Temperaturerhöhung des
Meßsensors eintritt, andererseits aber ein auswertbarer
Spannungsabfall am Meßsensor, als Maß für den Widerstand
und damit für die Temperatur, auftritt.

Das Ausgangssignal des Meßsensors 4 wird in einem
Verstärker 5 verstärkt und nach Kompensation der Einwirkung
der Umgebungstemperatur in einer Kompensationseinheit 6
einer Auswerteeinheit zugeführt, welche drei Komparatoren
7, 8, 9 aufweist. Die nachfolgende Zeitmeßeinheit 10
bestimmt die Länge der als Meßgröße dienenden Zeiten
$t_1$, $t_2$... .

Eine nachfolgende Recheneinheit 11 dient der Umrechnung der
Zeitwerte in die entsprechenden Werte der
Strömungsgeschwindigkeit bzw. der Stoffeigenschaften, welche
in einer Anzeigeeinheit 12 ausgelesen werden.

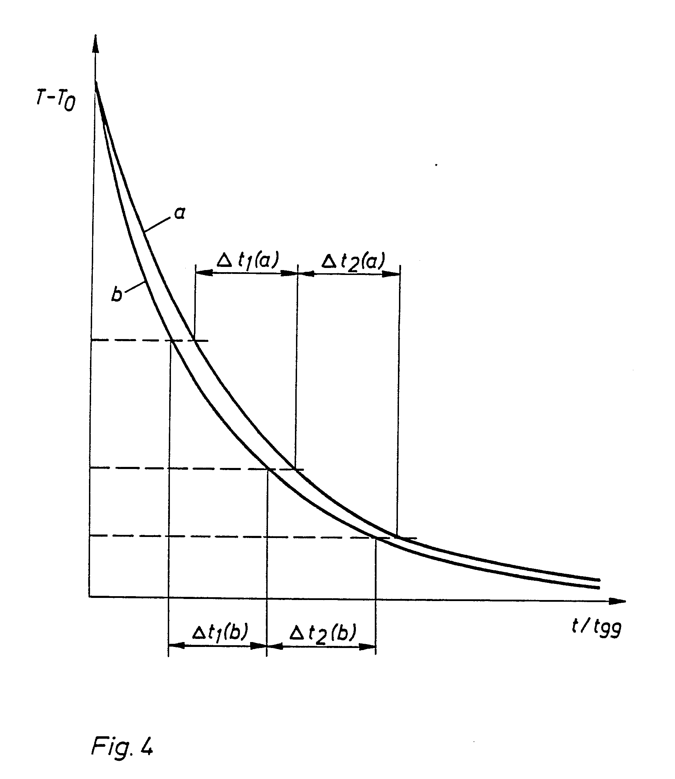

Fig. 3 zeigt das Abklingen der Aufheiztemperatur von $T-T_0$ auf
drei jeweils vorgegebene Temperaturwerte $T_1-T_0$, $T_2-T_0$, $T_3-T_0$,
wobei die hierzu erforderlichen Zeiten mit den Komparatoren
7, 8, 9 bestimmt werden. Die Komparatoren 7, 8 bestimmen die
Zeitdifferenz $\Delta t_1 = t_2 - t_1$, während die Komparatoren 8, 9
die Zeitdifferenz $\Delta t_2 = t_3 - t_2$ ermitteln.

Damit neben der Strömungsgeschwindigkeit in einem
strömungsfreien Intervall bei Nullwert der
Strömungsgeschwindigkeit auch zusätzliche Stoffeigenschaften
überprüft und bestimmt werden können, ist der zeitliche
Verlauf der Abkühlkurven zu überwachen: bei hohen
Strömungsgeschwindigkeiten ist das Abklingverhalten der
Abkühlkurven streng exponentiell, wie es die Abkühlkurve a
darstellt. Bei verschwindender Strömungsgeschwindigkeit
verhält sich die Abkühlkurve nach einem nicht-exponentiellen
Verlauf gemäß Abkühlkurve b (Fig. 4).

9

Als Maß für die Größe der Abweichung vom exponentiellen
Abklingen werden zwei Zeitdifferenzen $\Delta t_1$ und $\Delta t_2$ gemessen,
und zwar die Zeiten, die der Meßsensor benötigt, um von
einer Temperatur $T_1$ über der Umgebungstemperatur $T_0$ auf die
Temperatur $T_2 - T_0$ abzuklingen, wobei im exponentiellen
Verlauf gelten soll

$$K \cdot (T_1 - T_0) = T_2 - T_0$$

sowie weiter von der Temperatur $T_2 - T_0$ auf die Temperatur
$T_3 - T_0$ mit gleichem Koeffizienten K

$$T_3 - T_0 = K \cdot (T_2 - T_0) = K^2 \cdot (T_1 - T_0)$$

Für den Fall des exponentiellen Verlaufs (Kurve a) sind
die Zeiten $\Delta t_1$ (a) und $\Delta t_2$ (a) gleich groß. Ihr Verhältnis
ergibt sich zu 1. Bei Geschwindigkeiten im Bereich des
Nullwertes der Strömung treten unterschiedliche
Zeitdifferenzen auf, deren Quotient $\Delta t_1 : \Delta t_2$ überwacht werden
muß. Bei Erreichen eines experimentell bestimmbaren
Grenzwertes des Verhältnisses $\Delta t_1$ (b) $/ \Delta t_2$ (b) ist
anzunehmen, daß der Nullwert der Strömung vorliegt. Nachdem
dies festgestellt wurde, d.h. nachdem der Wärmeaustausch
zwischen dem Meßsensor und dem umgebenden Medium nur noch
von der Temperaturleitfähigkeit des Gases und damit von
Stoffeigenschaften, nicht aber von der durch eine Strömung
erzwungenen Konvektion abhängt, kann durch die Zeitmessung
für die Abkühlung auf eine vorgegebene Temperatur auf
Stoffeigenschaften geschlossen werden.

Eine zweckmäßige Ausführung des Meßverfahrens kann
beispielsweise vorsehen, daß nach mehreren Meßabschnitten
$M_1 \dots$ eine Abschaltung der Strömung des zu messenden Mediums
im Strömungskanal vorgenommen und der Nullwert der Strömung
in der angegebenen Weise unter Beachtung der Abweichung vom exponentiellen Abklingverhalten festgestellt wird. In einem daran anschließenden Meßabschnitt werden dann Stoffeigenschaften, beispielsweise die gleichbleibende Zusammensetzung einer Gasströmung, überprüft und gegebenenfalls für die weiter fortgesetzte Messung der Strömungsgeschwindigkeit rechnerisch berücksichtigt.

Eine zweckmäßige Anwendung des neuartigen Meßverfahrens zur Bestimmung des Strömungs-Nullwertes liegt u.a. in der Beatmungsüberwachung. In periodischen Strömungsvorgängen, wie sie bei der Beatmung auftreten, in denen regelmäßig Null-Geschwindigkeitsphasen erscheinen, kann durch routinemäßiges Überprüfen des Betrages der Abweichung vom exponentiellen Abklingverhalten der geschwindigkeitslose Zustand automatisch festgestellt werden. Dabei ist es möglich, eventuelle Änderungen in der Gaszusammensetzung mit zu erfassen.

Das Meßverfahren kann je nach den vorliegenden Gegebenheiten auch mit mehreren Meßsensoren von gleicher oder unterschiedlicher Ausbildung zweckmäßig durchgeführt werden.

Patentansprüche

1. Verfahren zur Bestimmung einer Meßgröße eines strömenden Mediums, insbesondere der Strömungsgeschwindigkeit und/oder der Stoffeigenschaften, bei dem die Temperaturänderung eines beheizten Meßsensors in einer elektronischen Meßschaltung zur Bestimmung der Meßgröße ausgewertet wird, dadurch gekennzeichnet, daß der Meßsensor innerhalb wenigstens eines Meßabschnittes mit einem Aufheizimpuls aufgeheizt wird, und daß als Meßgröße die Zeit bestimmt wird, nach der der Meßsensor auf mindestens eine vorgegebene Temperatur abgekühlt ist.

2. Verfahren nach Anspruch 1, dadurch gekennzeichnet, daß der Aufheizimpuls jeweils dann ausgelöst wird, wenn der Meßsensor eine vorgegebene Temperatur erreicht.

3. Verfahren nach Anspruch 1, dadurch gekennzeichnet, daß eine Reihe von Aufheizimpulsen in gleichem Abstand verwendet wird.

4. Verfahren nach Anspruch 1, dadurch gekennzeichnet, daß zur Bestimmung des Strömungs-Nullwertes die Größe der Abweichung vom exponentiellen Verlauf der Abkühlungs-Temperaturkurve festgestellt wird, und daß der Nullwert dann festgestellt wird, wenn der Betrag der Abweichung vom exponentiellen Verlauf seinen Maximalwert erreicht.

5. Verfahren nach Anspruch 4, dadurch gekennzeichnet, daß als Maß für die Größe der Abweichung vom exponentiellen Verlauf der Abkühlungs-Temperaturkurve Temperaturen $(T_1, T_2, T_3)$ gewählt werden, für die sich nach exponentiellem Verlauf gleiche Zeitdifferenzen $\Delta t_1 = t_2 - t_1$ und $\Delta t_2 = t_3 - t_2$ ergeben,

2

und daß das Verhältnis der tatsächlich gemessenen Zeitdifferenzen $\Delta t_1$ und $\Delta t_2$ zur Erreichung dieser Temperaturen $(T_1, T_2, T_3)$ bestimmt wird, wobei der Grenzwert dieses Verhältnisses die Geschwindigkeit Null der Strömung festlegt.

6. Verfahren nach Anspruch 1, dadurch gekennzeichnet, daß der Aufheizimpuls im Vergleich zur Länge des Meßabschnittes kurz ist.

7. Verfahren nach Anspruch 1, dadurch gekennzeichnet, daß die Aufheizzeit unter 150 $\mu$sec und die Längen der Meßabschnitte zwischen 1 - 50 $\mu$sec liegen.

8. Meßschaltung zur Durchführung des Verfahrens nach Anspruch 1, dadurch gekennzeichnet, daß ein elektronisches Schaltelement (2) vorgesehen ist, welches mit einem Pulsgenerator (1) verbunden ist und einen Meßsensor (4) mit temperaturabhängigem Widerstand in seiner einen Schaltstellung mit einer Heizspannungsquelle $(U_h)$ und in seiner anderen Schaltstellung mit einer Widerstandsmeßanordnung (3) verbindet, daß die Umschaltung zwischen beiden Schaltzuständen taktweise im gleichen Abstand bzw. dann erfolgt, wenn der Meßsensor einen vorgegebenen Widerstandswert erreicht, und daß zur Bestimmung des zeitlichen Abstands zwischen dem Aufheizimpuls und dem Erreichen des vorgegebenen Widerstandswertes Komparatoren (7,8,9) vorgesehen sind.

9. Meßschaltung nach Anspruch 8, dadurch gekennzeichnet, daß in Abhängigkeit von der Impulsfolge der Heizspannungsquelle jeweils eine Reihe von in gleichem Abstand liegenden Abstandsimpulsen erzeugt wird und daß die zu bestimmende Meßgröße durch Abzählen der Anzahl der Abstandsimpulse ermittelt wird, welche zwischen dem Aufheizimpuls und dem Erreichen des vorgegebenen Widerstandswertes liegt.

Fig.1

$T - T_0$

$T_1 - T_0$

$J_1$  $M_1$  $J_2$  $M_2$  $J_3$  $M_3$

$t_x$  $t_y$  $t_y$

$t$

Fig.3

$T - T_0$

$T_1 - T_0$
$T_2 - T_0$
$T_3 - T_0$

$t$

Komparator 7

$\Delta t_1$

Komparator 8

$\Delta t_2$

Komparator 9

$t_1$  $t_2$  $t_3$

Fig.2

Fig. 4

**Europäisches Patentamt**

# EUROPÄISCHER RECHERCHENBERICHT

Nummer der Anmeldung

EP 87 11 5372

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 300 227 (BATTELLE MEMORIAL INSTITUTE) <br> * Zusammenfassung; Seite 3, Zeile 31 - Seite 9, Zeile 8; Seite 9, Zeile 20 - Seite 10, Zeile 11; Figuren 1, 2, 4, 5 * | 1,2,6 | A 61 B 5/08 <br> G 01 F 1/68 <br> G 01 P 5/12 |
| A | --- | 4,5,8 | |
| A | DE-A-3 433 368 (BOSCH GMBH) <br> * Zusammenfassung; Seite 10, Zeile 27 - Seite 12, Zeile 7; Figuren 1, 2A, 2B * | 1,2,6,8 | |
| P,X | EP-A-0 210 509 (SCHMIDT FEINTECHNIK GMBH) <br> * Zusammenfassung; Seite 7, Zeile 5 - Seite 10, Zeile 4; Figuren 1, 2a, 2b * | 1-3 | |
| A | US-A-3 719 083 (MORRIS et al.) <br> * Spalte 1, Zeile 38 - Spalte 3, Zeile 29; Figuren 1, 2 * | 1 | |

**RECHERCHIERTE SACHGEBIETE (Int. Cl.4)**

A 61 B 5/00
G 01 F 1/00
G 01 P 5/12

Der vorliegende Recherchenbericht wurde für alle Patentansprüche erstellt

| Recherchenort | Abschlußdatum der Recherche | Prüfer |
|---|---|---|
| BERLIN | 10-02-1988 | WEIHS J.A. |

EPO FORM 1503 03.82 (P0403)